# EUROPÄISCHE PATENTSCHRIFT

(11) **EP 1 449 498 B1**
(45) Veröffentlichungstag und Bekanntmachung des Hinweises auf die Patenterteilung: **16.09.2009**
(21) Anmeldenummer: 04001985.3
(22) Anmeldetag: 29.01.2004
(51) Int. Cl.: A61F 2/16

(54) **Intraokularlinsen-Set**
Intraocular lens set
Jeu de lentilles intra-oculaires

(30) Priorität: 21.02.2003 DE 10307554
(43) Veröffentlichungstag der Anmeldung: 25.08.2004
(73) Patentinhaber: *Acri. Tec GmbH, 16761 Hennigsdorf (DE)
(72) Erfinder: Stork, Wilhelm, Dr., 76831 Impflingen (DE); Kreiner, Christine, Dr., 81545 München (DE)
(74) Vertreter: Nöth, Heinz

(56) Entgegenhaltungen:
- EP-A- 0 470 811
- EP-A- 0 563 783
- EP-A- 0 605 841
- WO-A-00/76426
- WO-A-01/04667
- US-A- 5 895 422

## Beschreibung

Die Erfindung betrifft ein Intraokularlinsen-Set nach dem Oberbegriff des Patentanspruches 1.

### [Stand der Technik]

Ein derartiges Intraokularlinsen-Set ist aus der EP 0 563 783 B1 bekannt. Das bekannte Intraokularlinsen-Set besitzt eine erste Intraokularlinse und eine zweite Intraokularlinse. Jede Linse ist bifokal ausgebildet, d.h. sie besitzt einen Nachfokus und einen Fernfokus. Die jeweilige Brechkraft für den Nahfokus und den Fernfokus sind aus einem diffraktiven und refraktiven Anteil der Linse zusammengesetzt. Die erste Linse weist für ihren Fernfokus einen höheren Anteil an Lichtverteilung auf als die zweite Linse. Die zweite Linse weist für ihren Nahfokus eine höhere Lichtverteilung auf als die erste Linse. Der Fernfokus der ersten Linse und der Nahfokus der zweiten Linse liegen auf der Retina der beiden Augen, in die die Linsen implantiert sind. Der refraktive Anteil wird beim bekannten Intraokularlinsen-Set an der Rückseite jeder Linse von einem diffraktiven Strukturprofil gebildet. Die Profilhöhe entspricht einer Wellenlänge im grünen Spektralbereich des sichtbaren Lichtes.

Bei der Entfernung von Nachstar an der Rückseite der implantierten Linse mit Hilfe von Laserbestrahlung besteht die Gefahr, dass die in unmittelbarer Nähe liegende diffraktive Struktur an der Linsenrückseite zerstört werden kann.

### [Aufgabe der Erfindung]

Aufgabe der Erfindung ist es, ein Intraokularlinsen-Set der eingangs genannten Art zu schaffen, bei dem die jeweilige Linse des Intraokularlinsen-Sets für eine gute Faltbarkeit dünn ausgebildet ist und die Gefahr der Zerstörung der diffraktiven Struktur bei der Nachstarbehandlung durch Laserstrahlen verringert ist.

Diese Aufgabe wird erfindungsgemäß durch die Merkmale des Patentanspruches 1 gelöst.

Bei der Erfindung besitzen die erste und die zweite Intraokularlinse an der jeweiligen vorderen Begrenzungsfläche eines zentralen Linsenbereichs eine zusammen mit dem refraktiven Anteil des zentralen Linsenbereiches den Nahfokus bildende diffraktive Struktur. Der zentrale Linsenbereich wird von einem ringförmigen Randbereich umgeben, welcher ringförmige monofokale diffraktive Zonen aufweist, die zusammen mit dem refraktiven Anteil die Basiskrümmung der beiden Begrenzungsflächen des zentralen Linsenbereiches den Fernfokus bilden. Der refraktive Anteil des zentralen Linsenbereiches wird von den Basiskrümmungen an der vorderen und hinteren Begrenzungsfläche und dem Material des zentralen Linsenbereiches bestimmt.

Die diffraktiven Zonen sowohl auf der Vorderseite des zentralen Linsenbereiches als auch die diffraktiven Zonen im Randbereich der Linse sind so bemessen, dass die optischen Weglängen einer diffraktiven Zone zur benachbarten diffraktiven Zone sich um eine Designwellenlänge oder um ein ganzzahliges Vielfaches der Designwellenlänge unterscheidet. Vorzugsweise wird die Designwellenlänge im grünen Spektralbereich des sichtbaren Lichtes ausgewählt. Es besteht daher Kohärenz des durch die diffraktiven Zonen des Randbereiches der Linse gehenden Lichts, welches zusammen mit dem refraktiven Anteil des zentralen Linsenbereiches den Fernfokus bildet. Ferner besteht Kohärenz für das durch die diffraktiven Zonen an der Vorderseite des zentralen Linsenbereiches hindurchgehenden Licht, welches im Nahfokus gebündelt ist. Die beiden Begrenzungsflächen des zentralen Linsenbereiches besitzen vorzugsweise eine asphärische Basiskrümmung. Der zentrale Linsenbereich besitzt vorzugsweise einen Durchmesser von etwa 4 mm ± 15 % und der ringförmige Randbereich eine Breite von etwa 1 mm ± 15 %. Vorzugsweise sind am ringförmigen Randbereich sowohl an der Vorderseite als auch an der Rückseite die diffraktiven Zonen vorgesehen.

Der zentrale Linsenbereich besitzt einen Radius, der etwa 2/3 des Gesamtradius des optischen Linsenteils entspricht. Der ringförmige Randbereich besitzt eine Breite, die etwa einem Drittel des Gesamtradius des optischen Linsenteils entspricht.

Die unterschiedliche Lichtverteilung bei den beiden Intraokularlinsen für das aus dem Fernbereich kommende Licht im Fernfokus und für das aus dem Nahbereich kommende Licht im Nahfokus kann mittels der Beugungseffizienz der diffraktiven Struktur auf der Vorderseite des zentralen Linsenbereiches sowie der diffraktiven Zonen im Randbereich entsprechend eingestellt werden. Dies wird durch die geometrische Gestaltung der diffraktiven Struktur, nähmlich deren Stufenhöhe und Stufenbreite erzielt. Hierdurch lässt sich eine Variation der Beugungseffizienz zwischen 10 % bis 90 % und damit eine entsprechende Einstellung der Lichtverteilung auf den jeweiligen Nahfokus und Fernfokus erzielen.

### [Beispiele]

Anhand der Figuren wird die Erfindung noch näher erläutert.

Es zeigt
- Fig. 1: eine schnittbildliche Darstellung einer Linsenhälfte in einem die Linsendicke und den Linsenradius angebenden Koordinationssystem; und
- Fig. 2: die beiden Linsen des Intraokularlinsen-Sets mit den zugehörigen Lichtverteilungen.

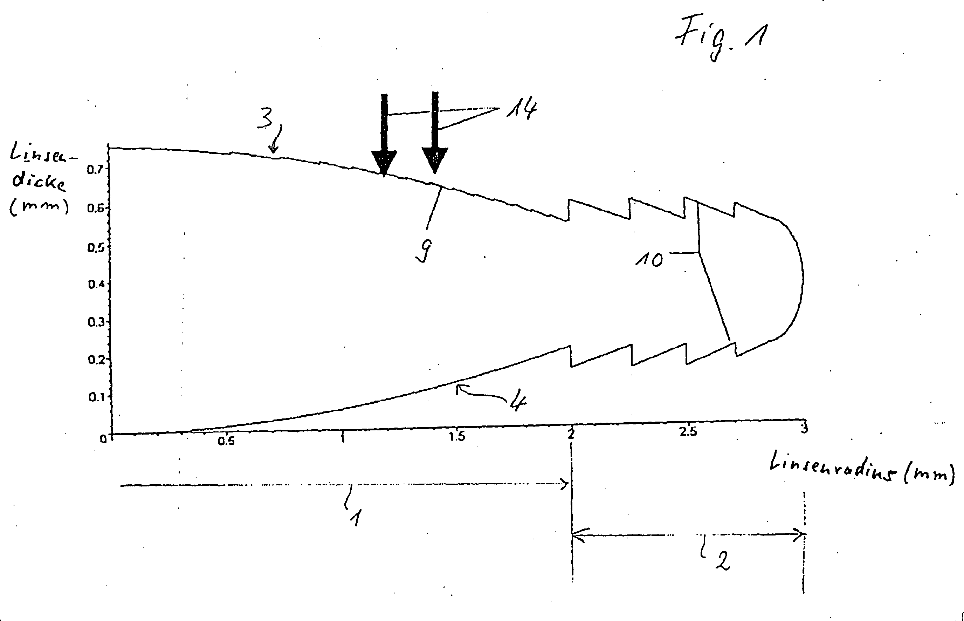

Die in Fig. 1 dargestellte Intraokularlinse besitzt einen zentralen Linsenbereich 1 mit einem Durchmesser von etwa 4 mm. Der zentrale Linsenbereich 1 wird von einem ringförmigen Randbereich 2 umgeben. Der ringförmige Randbereich besitzt eine ringförmige diffraktive Zone 10 mit etwa Sägezahnstruktur. Wie aus der WO 00/76426 bekannt ist, unterscheiden sich die optischen Weglängen des durch die diffraktiven Zonen 10 hindurchgegangenen Lichts um ein ganzzahliges Vielfaches der Designwellenlänge. Vorzugsweise entspricht die Profilhöhe der diffraktiven Zonen 10 einer Wellenlänge im grünen Spektralbereich des sichtbaren Lichts. Das durch die diffraktiven Zonen 10 im Randbereich 2 der Linse hindurchgehende Licht ist kohärent. Die monofokalen diffraktiven Zonen 10 des Randbereiches 2 wirken mit dem refraktiven Teil des zentralen Linsenbereiches 1 zur Bildung des Fernfokus zusammen. Der zentrale Linsenbereich 1 besitzt an der Vorderseite und an der Rückseite Begrenzungsflächen 3 und 4 mit vorzugsweise asphärischer Basiskrümmung, welche bei vorgegebenem Linsenmaterial zur Einstellung des refraktiven Anteils des zentralen Linsenbereiches 1 dienen.

Auf der Vorderseite des zentralen Linsenbereiches 1 befinden sich ringförmige diffraktive Zonen 9, welche zusammen mit dem refraktiven Anteil des zentralen Linsenbereiches 1 einen Nahfokus 7 der Linse bilden. Die Breite des Randbereiches 2 entspricht etwa einem Drittel des Radius des optischen Teils der Linse. Bei einem Radius des optischen Linsenteils von 3 mm hat der Randbereich 2 eine Breite von etwa 1 mm ± 15 % und der Radius des zentralen Linsenbereiches beträgt etwa 2 mm ± 15 %.

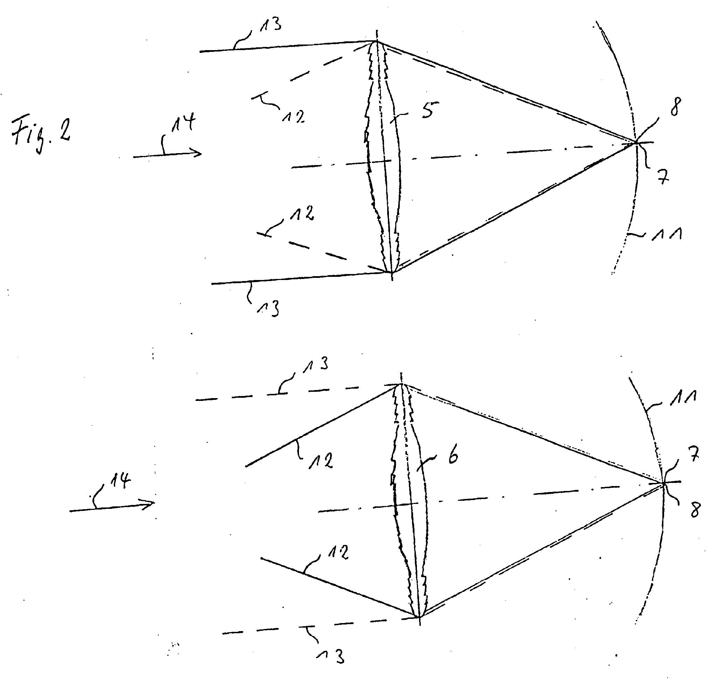

In der Fig. 2 sind die beiden Linsen 5 und 6 des Intraokularlinsen-Sets mit den Lichtverteilungen für den jeweiligen Nahfokus 7, in welchem das aus dem Nahbereich kommende Licht 12 gesammelt wird, und den jeweiligen Fernfokus 8, in welchem das aus der Ferne kommende Licht 13 gesammelt wird, dargestellt. Die erste Linse 5 besitzt für den auf einer Retina 11 eines Auges liegenden Fernfokus 8 einen höheren Anteil an Lichtverteilung als der Nahfokus 7 dieser Linse. Der höhere Anteil der Lichtverteilung für den Fernfokus 8 liegt vorzugsweise bei 60 % bis 70 % oder mehr der Gesamtlichtverteilung. Die zweite Intraokularlinse 6 besitzt für den auf der Retina 11 des anderen Auges liegenden Nahfokus 7 einen höheren Anteil an Lichtverteilung als der Fernfokus 8. Der höhere Anteil der Lichtverteilung auf der Retina 11 liegenden Nahfokus 7 kann ebenfalls 60 % bis 70 % oder mehr der Gesamtlichtverteilung, welche durch die Linse 6 hindurchtritt, betragen. In der Fig. 2 ist mit durchgezogenen Linien der jeweils höhere Anteil der Lichtverteilung und mit strichlierten Linien der jeweils geringere Anteil der Lichtverteilung dargestellt. Das aus der Ferne kommende Licht 13 wird im Fernfokus 8 und das aus dem Nahbereich kommende Licht 12, beispielsweise von Gegenständen, die im Abstand von etwa 30 bis 40 cm vom Auge entfernt sind, im Nahfokus 7 jeweils auf der Retina 11 gesammelt. Die Brennweiten für die Nah- und Fernfoki 7 und 8 entsprechen daher im wesentlichen den Abständen der implantierten Linsen 5 und 6 von den Retinae der beiden Augen.

Mit der Vorderseite der Linse ist die Seite der Linse bezeichnet, auf welche im implantierten Zustand das von außen kommende Licht 14 auftrifft. Die Rückseite der Linse ist im implantierten Zustand die der Retina zugewandte Linsenseite.

### [Bezugszeichenliste]

- 1: zentraler Linsenbereich
- 2: ringförmiger Randbereich
- 3: vordere Begrenzungsfläche des zentralen Linsenbereiches
- 4: hintere Begrenzungsfläche des zentralen Linsenbereiches
- 5: erste Intraokularlinse
- 6: zweite Intraokularlinse
- 7: Nahfokus
- 8: Fernfokus
- 9: diffraktive Struktur auf der Vorderseite des zentralen Linsenbereiches
- 10: diffraktive Zonen im Randbereich
- 11: Retina
- 12: aus dem Nahbereich kommendes Licht
- 13: aus der Ferne kommendes Licht
- 14: von außen in das Auge einfallende Licht

## Patentansprüche

1. lntraokularlinsen-Set mit einer ersten Intraokularlinse und einer zweiten Intraokularlinse, von denen jede Linse einen Nahfokus und einen Fernfokus aufweist und bei denen die jeweilige Brechkraft aus einem diffraktiven und einem refraktiven Anteil zusammengesetzt sind, wobei die erste Linse für ihren Fernfokus einen höheren Anteil an Lichtverteilung hat als die zweite Linse und die zweite Linse für ihren Nahfokus eine höhere Lichtverteilung hat als die erste Linse,
**dadurch gekennzeichnet, dass** die erste und zweite Linse (5, 6) jeweils an ihrer Vorderseite an einer Begrenzungsfläche (3) eines zentralen Linsenbereichs eine den Nahfokus (7) bildende diffraktive Struktur (9) aufweist und der zentrale Linsenbereich (1) von einem ringförmigen Randbereich (2) umgeben ist, welcher ringförmige monofokale diffraktive Zonen (10) aufweist, die zusammen mit der Basiskrümmung der beiden Begrenzungsflächen (3, 4) des zentralen Linsenbereiches (1) den Fernfokus (8) bilden, wobei die unterschiedliche Lichtverteilung durch die Stufenhöhe und Stufenbreite der diffraktiven Strukturen (9, 10) auf der Vorderseite des zentralen Linsenbereichs (1) und auf dem den zentralen Linsenbereich (1) umgebenden ringförmigen Randbereich (2) eingestellt ist.

2. Intraokularlinsen-Set nach Anspruch 1,
**dadurch gekennzeichnet, dass** die optischen Weglängen einer diffraktiven Zone (9, 10) zur benachbarten diffraktiven Zone an der vorderen Begrenzungsfläche (3) des zentralen Linsenbereiches (1) und im Randbereich (2) der Linse (5, 6) sich um eine Designwellenlänge oder um ein ganzzahliges Vielfaches der Designwellenlänge unterscheiden.

3. Intraokularlinsen-Set nach Anspruch 1 oder 2,
**dadurch gekennzeichnet, dass** wenigstens eine der beiden Begrenzungsflächen (3, 4) des zentralen Linsenbereiches eine asphärische Basiskrümmung aufweist.

4. Intraokularlinsen-Set nach einem der Ansprüche 1 bis 3,
**dadurch gekennzeichnet, dass** die Strukturhöhe der jeweiligen diffraktiven Zone auf eine Designwellenlänge im grünen Spektralbereich des sichtbaren Lichts bemessen ist.

5. Intraokularlinsen-Set nach einem der Ansprüche 1 bis 4, **dadurch gekennzeichnet, dass** der zentrale Linsenbereich (1) einen Radius von etwa 2/3 des Gesamtradius des optischen Linsenteils und der ringförmige Randbereich eines Breite, die etwa einem Drittel des Gesamtradius des optischen Linsenteils entspricht, aufweisen.

6. Intraokularlinsen-Set nach einem der Ansprüche 1 bis 5,
**dadurch gekennzeichnet, dass** im ringförmigen Randbereich (2) auf der Vorderseite und Rückseite der Linse (5, 6) diffraktive Zonen (10) vorgesehen sind.

## Claims

1. Intraocular lens set comprising a first intraocular lens and a second intraocular lens, each of which having a near focus and a far focus, the respective refraction force of said lenses consists of a diffractive and a refractive portion, wherein the first lens has a greater portion of light distribution for its far focus than the second lens, and the second lens has a greater portion of light distribution for its near focus than the first lens,
**characterized in that** the first and second lenses (5, 6) have respectively at their front side at a boundary face (3) of a central lens area a diffractive structure (9) forming the near focus (7), and that the central lens area (1) is surrounded by an annular border area (2) having annular monofocal diffractive zones (10) which form the far focus (8) in combination with the basic curvature of both boundary faces (3, 4) of the central lens area (1), wherein the different light distribution is adjusted by the step height and the step width of the diffractive structures (9, 10) at the front side of the central lens area (1) and at the annular border area (2) surrounding the central lens area (1).

2. lntraocular lens set according to claim 1,
**characterized in that** the optical path lengths of a diffractive zone (9, 10) to a adjacent diffractive zone at the front boundary face (3) of the central lens area (1) and in the border area (2) of the lens (5, 6) differ from each other by a design wavelength or by an integer multiple of the design wavelength.

3. Intraocular lens set according to claim 1 or 2,
**characterized in that** at least one of the two boundary faces (3, 4) of the central lens area has a aspherical basic curvature.

4. lntraocular lens set according to any one of claims 1 to 3,
**characterized in that** the structure height of the respective diffractive zone is determined for a design wavelength in the green spectral region of visible light.

5. Intraocular lens set according to any one of claims 1 to 4,
**characterized in that** the central lens area (1) has a radius of approximately 2/3 of the overall radius of the optical lens portion, and the annular border area has a width corresponding to approximately one third of the overall radius of the optical lens portion.

6. Intraocular lens set according to any one of claims 1 to 5,
**characterized in that** diffractive zones (10) are provided in the annular border area (2) at the front side and at the rear side of the lens (5, 6).

## Revendications

1. Jeu de lentilles intraoculaires, comprenant une première lentille intraoculaire et une seconde lentille intraoculaire, parmi lesquelles chaque lentille présente un foyer proche et un foyer éloigné et dans lesquelles le pouvoir réfringent respectif est composé d'une fraction diffractive et d'une fraction réfractive, la première lentille ayant, pour son foyer éloigné, une fraction de diffusion lumineuse plus élevée que celle de la seconde lentille et la seconde lentille ayant, pour son foyer proche, une diffusion lumineuse plus élevée que celle de la première lentille,
**caractérisé en ce que** la première et la seconde lentilles (5, 6) présentent, respectivement sur leur face avant, sur une surface de délimitation (3) d'une zone de lentille centrale, une structure diffractive (9) formant le foyer proche (7) et la zone de lentille centrale (1) est entourée par une zone de bord annulaire (2), qui présente des zones diffractives monofocales annulaires (10) qui, conjointement avec la courbure de base des deux surfaces de délimitation (3, 4) de la zone de lentille centrale (1), forment le foyer éloigné (8), la diffusion lumineuse différente étant réglée par la hauteur d'échelon et la largeur d'échelon des structures diffractives (9, 10) sur la face avant de la zone de lentille centrale (1) et sur la zone de bord annulaire (2) entourant la zone de lentille centrale (1) .

2. Jeu de lentilles intraoculaires selon la revendication 1,
**caractérisé en ce que** les trajets optiques d'une zone diffractive (9, 10) jusqu'à la zone diffractive voisine sur la surface de délimitation avant (3) de la zone de lentille centrale (1) et dans la zone de bord (2) de la lentille (5, 6) diffèrent d'une longueur d'onde théorique ou d' un multiple entier de la longueur d'onde théorique.

3. Jeu de lentilles intraoculaires selon la revendication 1 ou 2,
**caractérisé en ce qu'**au moins l'une des deux surfaces de délimitation (3, 4) de la zone de lentille centrale présente une courbure de base asphérique.

4. Jeu de lentilles intraoculaires selon l'une quelconque des revendications 1 à 3,
**caractérisé en ce que** la hauteur de structure de la zone diffractive respective est dimensionnée sur une longueur d'onde théorique dans la zone spectrale verte de la lumière visible.

5. Jeu de lentilles intraoculaires selon l'une quelconque des revendications 1 à 4,
**caractérisé en ce que** la zone de lentille centrale (1) présente un rayon d'environ les 2/3 de la valeur du rayon total de la partie de lentille optique et la zone de bord annulaire présente une largeur qui correspond environ à un tiers du rayon total de la partie de lentille optique.

6. Jeu de lentilles intraoculaires selon l'une quelconque des revendications 1 à 5,
**caractérisé en ce qu'**il est prévu des zones diffractives (10) dans la zone de bord annulaire (2) sur la face avant et la face arrière de la lentille (5, 6) .
